# EUROPÄISCHE PATENTSCHRIFT

(11) **EP 1 649 889 B1**
(45) Veröffentlichungstag und Bekanntmachung des Hinweises auf die Patenterteilung: **10.09.2008**
(21) Anmeldenummer: 05022550.7
(22) Anmeldetag: 15.10.2005
(51) Int. Cl.: A61M 25/00

(54) **Katheter, insbesondere zur Einführung von Herzschrittmacher-oder ICD-Elektroden in einen Patientenkörper**
Catheter particularly for insertion of pace-maker or ICD-electrodes into a patient's body
Cathéter particulièrement pour insertion de pace-maker ou d'électrode DCI dans le corps d'un patient

(30) Priorität: 20.10.2004 DE 102004051211
(43) Veröffentlichungstag der Anmeldung: 26.04.2006
(73) Patentinhaber: BIOTRONIK CRM Patent AG, 6340 Baar (CH)
(72) Erfinder: Flach, Erhard, 12305 Berlin (DE); Geistert, Wolfgang, Dr., 79618 Rheinfelden (DE); Maxfield, Michelle, 10967 Berlin (DE)
(74) Vertreter: Lindner-Vogt, Karin L.

(56) Entgegenhaltungen:
- EP-A- 0 608 853
- EP-A- 0 790 066
- EP-A- 1 457 224
- WO-A-96/38193
- US-A- 5 507 751
- US-A- 5 743 876
- US-B1- 6 251 092
- PATENT ABSTRACTS OF JAPAN Bd. 2002, Nr. 02, 2. April 2002 (2002-04-02) & JP 2001 299923 A (MITSUBISHI CABLE IND LTD), 30. Oktober 2001 (2001-10-30) -& DATABASE WPI Derwent Publications Ltd., London, GB; AN 2002-031204 XP002364398 & JP 2001 299923 A (MITSUBISHI CABLE IND LTD) 30. Oktober 2001 (2001-10-30)

## Beschreibung

Die Erfindung betrifft einen Katheter insbesondere zur Einführung von Herzschrittmacher- oder ICD-(= Implantible cardioverter defibrillator-) Elektroden in einen Patientenkörper mit einer Katheterwand und einer Verstärkung darin.

Derartige Katheter umfassen bekanntermaßen mindestens ein Lumen, das von der Katheterwand umgrenzt wird und zur Führung der nach dem Einschieben des Katheters in den Patientenkörper einzusetzenden Elektrode dient. Über ein weiteres Lumen kann der Katheter beispielsweise über einen vorher im Patientenkörper applizierten Führungsdraht geschoben oder mit einem Zugdraht für die Katheterspitze ausgerüstet werden.

Ferner ist es bekannt, dass derartige Katheter entlang ihrer Längsaxialrichtung eine schlitz- oder aufreißbare Katheterwand aufweisen, so dass sie nach Platzierung der Elektrode im Patientenkörper einfach davon entfernt werden können. Einen solchen Katheter zeigt die EP 0 655 257 A2, die eine Schwächungslinie in Form einer Perforation in der Katheterwand offenbart.

Ein gängiges Problem bei solchen Kathetern liegt schließlich in der Torsions- und Knickstabilität des Katheterschaftes und dessen Kompressionsempfindlichkeit, insbesondere wenn der Katheter in einem kleinen Radius gebogen werden muss, wie die beispielsweise beim Einführen in die Arteria subclavia der Fall ist.

Aus dem Stand der Technik ist es zur Lösung dieser Problematik bereits bekannt, Verstärkungen in der Katheterwand vorzusehen, mit deren Hilfe der Katheter bezüglich seiner Torsions- und/oder Knicksteifigkeit und gegen ungewünschte Komprimierung stabilisiert wird. Die EP 0 898 481 B1, US 6 159 198 A und WO 99/33509 A1 offenbaren in diesem Zusammenhang die Einlage von Verstärkungsfasern in die Katheterwand. Diese können als Einzelfasern, wie in der WO 99/33509 A1 und der EP 0 898 481 B1 gezeigt, oder als Netzschlauch angelegt sein, wie dies aus der US 6 159 198 A hervorgeht.

Dokument US-A-5 743 876 zeigt einen Katheter, unter anderem zum Einführen von Herzschrittmacher- oder ICD-Elektroden in einen Patientenkörper, der eine Katheterwand, die mindestens ein Lumen umgrenzt, und ein in der Katheterwand befindliches Profilteil umfasst. Auf zumindest einer Teillänge ist das Profilteil als Gitterprofil mit Axial- und/oder Peripherstegen ausgebildet, um den Katheter bezüglich seiner Torsions-, Biege-, Kompressibilität- und/oder Knicksteifigkeit zu stabilisieren und bezüglich dessen mechanischer Eigenschaften zu bestimmen.

Dokument US-A-4 451 256 offenbart einen Schlauch zur Einführung eines Katheters, worin eine longitudinale aufteilbare Region zur Entfernung des Schlauches vom Katheter durch die Aufteilung der Region dient.

Wenngleich durch die eingelegten Fasern das Ziel einer Verstärkung grundsätzlich erreichbar ist, verursachen derartige Fasereinlagen einige Probleme insbesondere hinsichtlich der Katheterfertigung. Zwar ist die Einlage von Faserverstärkungen in Form einer Netz- oder Litzenstruktur in Schlauchgebilde grundsätzlich ein übliches Produktionsverfahren, jedoch können solche Faserverstärkungen nur schwer in Axial- und Peripherrichtung an bestimmte Solleigenschaften angepasst werden. Dies trifft insbesondere auch für die Schwächung des Katheters zur Bildung einer Aufreißlinie zu. Die erwähnte EP 0 898 481 B1 zeigt in diesem Zusammenhang eine Perforationslinie, die die in Peripherrichtung in der Katheterwand verlaufenden Verstärkungsfasern jeweils nur anreißt. Eine Umsetzung dieser dort schematisch dargestellten Maßnahme in die Praxis dürfte nur mit erheblichem Fertigungsaufwand möglich sein. Dies trifft umso mehr auf die ebenfalls in der vorgenannten Druckschrift gezeigten Verstärkungsfasern mit stark flach-rechteckigem Querschnitt zu, bei denen bei zu tiefen Ansetzen der Perforationslinien eine Durchtrennung der Verstärkungsfasern zu befürchten ist.

Ausgehend von den geschilderten Problemen des Standes der Technik liegt der Erfindung die Aufgabe zugrunde, einen Katheter so zu verbessern, dass auf einfache Weise sowohl in axialer als auch in peripherer Richtung wechselnde mechanische Eigenschaften, wie Torsions-, Biege-, Knickstabilität, Kompressibilität, Flexibilität und Schlitzbarkeit erzeugbar sind.

Die Lösung dieser Aufgabe ist im Kennzeichnungsteil des Anspruches 1 angegeben und sieht in ihrer grundsätzlichen Ausprägung vor, die Verstärkung als Profilteil auszubilden, das in Axial- und Peripherrichtung des Katheters auf dessen mechanische Solleigenschaften abgestimmt ist. So kann beispielsweise durch die sich ändernde Wandstärke des Profilteils eine über die Länge des Katheters wechselnde Flexibilität, Knicksteifigkeit und Kompressionsfestigkeit erzielt werden. Es ist also vorteilhafterweise nicht mehr notwendig - wie dies ebenfalls aus dem Stand der Technik bekannt ist - zur Erzeugung in axialer und/oder peripherer Richtung wechselnder Eigenschaften verschiedene unterschiedliche Katheterschaft-Stücke aneinander zu setzen und miteinander zu verkleben oder zu verschweißen. Eine Schlitzbarkeit des Katheters kann z. B. durch eine bezogen auf die Peripherrichtung vorgenommene Wanddickeneinschnürung des Profilteils hergestellt werden.

Bevorzugte Ausführungsformen der Erfindung sind in den Unteransprüchen angegeben. Weitere Merkmale, Einzelheiten und Vorteile der Erfindung gehen im Übrigen aus der folgenden Beschreibung hervor, in der Ausführungsbeispiele anhand der beigefügten Zeichnungen näher erläutert werden. Es zeigen:
- Fig. 1: eine höchst schematische, ausschnittsweise und teilweise weggebrochene Perspektivdarstellung eines Katheters in einer ersten Ausführungsform,
- Fig. 2 bis 4: Perspektivdarstellungen analog Fig. 1 dreier weiterer Ausführungsformen des Katheters,
- Fig. 5: eine schematische ausschnittsweise Perspektivdarstellung eines in den Katheter gemäß Fig. 4 eingesetzten Profilteils.

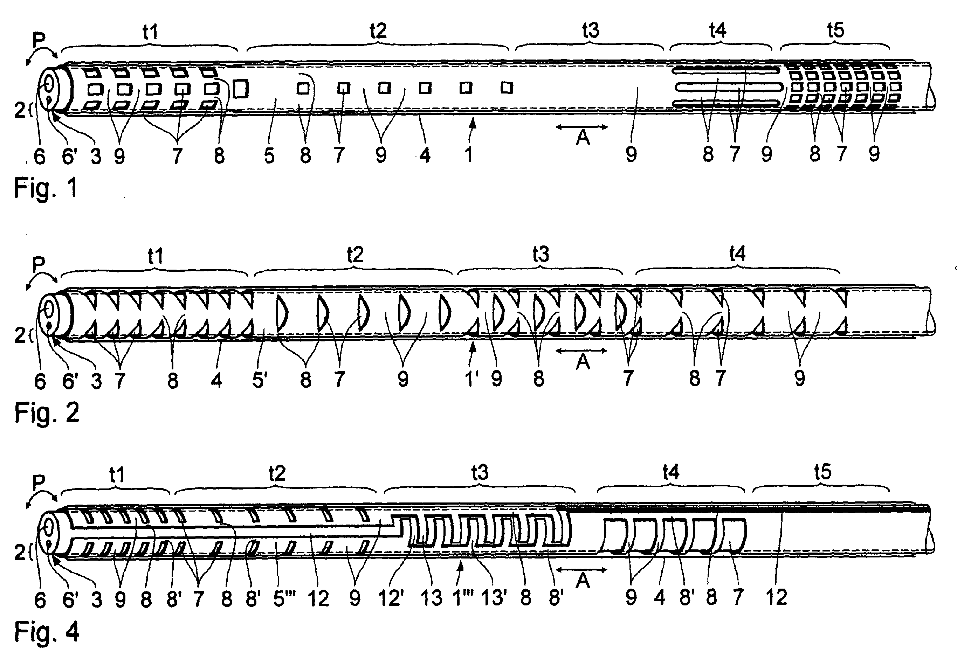

Der in Fig. 1 gezeigte Katheter 1 weist eine Katheterwand 2 auf, die radial von innen nach außen durch einen Innenschlauch 3, einen Außenschlauch 4 und ein dazwischen liegendes Profilteil 5 aufgebaut ist. Im Innenschlauch 3 sind zwei vom proximalen zum distalen Ende (jeweils nicht dargestellt) des Katheters 1 verlaufende Lumen 6, 6' angelegt, durch die beispielsweise eine Herzschrittmacher-Elektrode und ein Zugdraht durchschiebbar sind. Innen- und Außenschlauch 3, 4 bestehen aus einem extrudierten Elastomermaterial, wie es zur Anwendung in medizinischen Schläuchen und Röhren gängig ist.

Das Profilteil 5 ist in dem in Fig. 1 gezeigten Ausführungsbeispiel als in der Grundform längszylindrisches Gitterprofilteil ausgebildet, das in dem dargestellten Bereich in Teillängen t1, t2, t3, t4 und t5 mit unterschiedlicher Profilierung gegliedert ist. Grundsätzlich sind durch Öffnungen 7 in seiner Mantelwand in Axialrichtung A verlaufende Axialstege 8 und in Peripherrichtung P verlaufende Peripherstege 9 gebildet. Wie in der Zeichnung angedeutet ist, kann durch eine Variierung der Dicke der Mantelwand des Gitterprofilteils 5, sowie insbesondere der Größe der Öffnungen 7 und/oder der Querschnittsdimensionierung der Axial- und Peripherstege 8, 9 das Profilteil in unterschiedlichen Teillängen t1 bis t5 so ausgelegt werden, dass es auf die mechanischen Solleigenschaften des Katheters abgestimmt ist. Beispielsweise kann durch eine Vergrößerung der Öffnungen 7 jeweils in Richtung zum distalen Ende hin unter entsprechender Verringerung der Breiten der Axial- und Peripherstege 8, 9 der Torsions- und Biegewiderstand des Katheters 1 auf der Teillänge t1 gegenüber der Teillänge t2 deutlich verringert werden. Ferner können ― wie nicht explizit dargestellt ist - zwischen zwei Reihen von Öffnungen 7 nur sehr dünne und kurze Peripherstege 9 stehen gelassen werden, wodurch diese dann eine Sollbruchstelle zum Auftrennen des Katheters 1 und dessen Abnehmen von der eingesetzten Elektrode bilden. Auch kann eine "Mischform" von Axial- und Peripherstegen durch schräg verlaufende Diagonalstege realisiert werden.

Auf der Teillänge t3 ist das Profilteil nicht mit Öffnungen 7 versehen, also zylindrisch ausgeführt, wodurch es sehr kompressionsstabil ist.

Es schließt sich auf der Teillänge t4 ein Bereich langgestreckter Öffnungen 7 an, die zwischen sich zueinander parallel Axialstege 8 bilden. In diesem Bereich ist das Profilteil 5 relativ torsionsinstabil, kann also leicht in sich verdrillt werden.

Auf der Teillänge t5 schließlich ist ein relativ enges Gittermuster von Axial- und Peripherstegen 8, 9 durch enggesetzte Öffnungen 7 angelegt, das das Profilteil 5 in diesem Teil sehr biegsam, allerdings torsionsstabil macht.

Das Gitterprofilteil 5 gemäß Fig. 1 kann auf verschiedene Art und Weise hergestellt werden. So ist ein Laserschneiden, Stanzen oder Ätzen aus einem Schlauch, Röhrchen oder Flachmaterial, wie einem Folienstück möglich. Im letztgenannten Fall wird das im flachen Zustand profilierte Materialstück anschließend gerollt und als Profilteil beispielsweise auf den Innenschlauch 3 gewickelt. Auch eine Herstellung als spritzgegossener, extrudierter oder ähnlich heißverformter Schlauch oder Röhrchen ist denkbar.

Die vorstehenden Halbzeuge können aus Kunststoffmaterial, wie Polycarbonat, Polyimid und dergleichen oder aus einem Metallmaterial, wie Nitinol oder Edelstahl hergestellt sein.

Das so als Halbzeug hergestellte Gitterprofilteil 5 kann nach seiner Herstellung durch Kleben, Schrumpfen oder Schmelzen zwischen den Innen- und Außenschlauch 3, 4 eingebettet werden. Auch ein direktes Einspritzen in eine einstückige Schlauchwandung ist möglich.

Neben den vorstehenden mechanischen und thermoplastischen Herstellungstechniken kann das Gitterprofilteil 5 auch durch Beschichten des Innenschlauches 3 mit einem in UV- oder Laserlicht oder auch chemisch aushärtbaren Kunststoff durch eine Art lithografisches Verfahren hergestellt werden. Dazu wird auf die Beschichtung eine Maske gelegt, welche ein Negativ der gewünschten Gitterprofilierung darstellt. Durch Bestrahlung oder In-Kontakt-Bringen mit einem chemischen Härter ― etwa durch Tauchen oder Besprühen ― wird die Beschichtung an den exponierten, von der Maske freien Stellen durch Vernetzung ausgehärtet und anschließend die Maske entfernt. Dann können die nicht-ausgehärteten Zonen der Beschichtung mit einem geeigneten Lösungsmittel ausgewaschen werden und das Gitterprofilteil 5 mit dem gewünschten Lochmuster bleibt zurück. Der Außenschlauch 4 wird dann über das so hergestellte Profilteil 5 mit Innenschlauch 3 geschrumpft, geklebt oder extrudiert.

Die vorstehende Maskierung kann dann entfallen, wenn die Beschichtung mit einem Scannerlaser oder fokusierten UV-Licht in den auszuhärtenden Zonen abgescannt und dort vernetzt wird.

Schließlich kann das Profilteil 1 auch durch Bedrucken des Innenschlauches 3 mit einem "Stegbild" aus einem aushärtbaren Material wie einer entsprechenden Druckpaste, und anschließendem Aushärten des Materials durch Strahlungs- oder chemische Vernetzung hergestellt werden.

Bei der in Fig. 2 gezeigten Ausführungsform des Katheters 1' sind wiederum ein Innenschlauch 3 mit zwei Lumen 6, 6', ein Außenschlauch 4 und ein dazwischen sitzendes Profilteil 5' vorgesehen. Letzteres ist mit einer von der Ausführungsform gemäß Fig. 1 abweichenden Profilierung auf seinen Teillängen t1 bis t4 versehen. Charakteristisch bei dieser Ausführungsform ist die Auslegung des Profilteils 5' dahingehend, dass der Katheter 1' auf diesen Teillängen t1 bis t4 definiert in bestimmten Ebenen besonders gut biegbar ist. So sind auf der Teillänge t1 auf zwei einander abgewandten Seiten aneinandergereihte, zwickelförmige Öffnungen 7 vorgesehen, die zwischen sich Peripherstege 9 mit sanduhrförmigem Breitenverlauf bilden. Die Peripherstege 9 sind durch zwei gegenüberliegende Axialstege 8 miteinander verbunden. Durch diese Konfiguration ist der Katheter auf der Teillänge t1 besonders gut in der Zeichnungsebene der Fig. 2 biegbar.

Auf der Teillänge t2 sind die zwickelförmigen Öffnungen 7 um 90° versetzt gegenüber denjenigen auf der Teillänge t1 und mit größerem Abstand zueinander angeordnet. Dadurch ist der Katheter auf dieser Teillänge t2 in der Zeichnungsebene der Fig. 2 steif, jedoch für eine Auslenkbewegung aus der Zeichnungsebene gut biegbar. Aufgrund des größeren Abstandes der Öffnungen 7 gegenüber Teillänge t1 ist die Biegbarkeit insgesamt jedoch geringer.

Auf der Teillänge t3 sind aufeinanderfolgende Öffnungen 7 jeweils um 90° versetzt zueinander, sodass eine Art Schräggitter-Profilteil mit schrägverlaufenden Peripherstegen 9 und in Axialrichtung unterbrochenen Axialstegen 8 gebildet wird. Durch den 90°-Versatz aufeinanderfolgender Öffnungen 7 ist der Katheter 1' auf dieser Teillänge t3 flexibel sowohl in der Zeichnungsebene der Fig. 2 als auch senkrecht dazu.

Auf der Teillänge t4 sind noch wenige Öffnungen 7 mit großem Abstand zueinander vorgesehen, sodass im dortigen Bereich der Katheter 1' sehr steif ist und allenfalls eine geringe Biegbarkeit in der Zeichnungsebene der Fig. 2 (analog Teillänge t1) zeigt.

Hinsichtlich der Herstellung und Einbindung des Profilteils 5' in den Katheteraufbau kann auf die Ausführungen zur Fig. 1 zurückgegriffen werden.

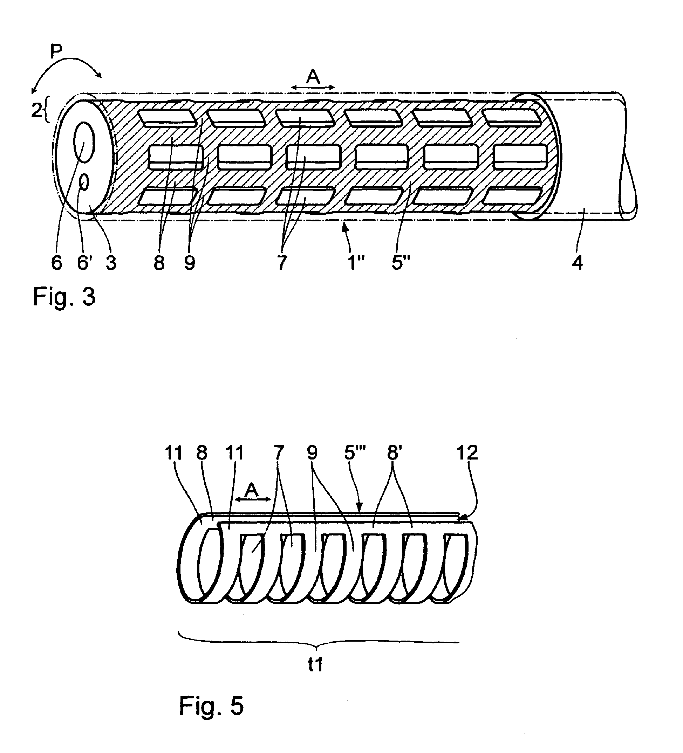

Bei der in Fig. 3 dargestellten Ausführungsform des Katheters 1" wird die Gitterstruktur des Profilteils 5" direkt im Innenschlauch 3 angelegt. Letzterer weist im gezeigten Fall wiederum zwei Lumen 6, 6' auf und ist an seiner Oberfläche mit einer in Fig. 2 schraffiert hervorgehobenen, insgesamt eine Gitterstruktur mit Axial- und Peripherstegen 8, 9 bildenden Verstärkung versehen. Dazu wird für den Innenschlauch 3 ein aushärtbares Material verwendet, das in den in Fig. 3 schraffierten Zonen bestrahlt und dadurch ausgehärtet wird. Ein Entfernen der nicht bestrahlten Zonen kann unterbleiben, so dass trotz der integrierten Verstärkung das Bauteil eine ebene Oberfläche aufweist. Um eine weitere Aushärtung des Innenschlauchs 3 und damit eine weitergehende Verstärkung durch Wachsen der Stege 8, 9 des Profilteils 5" zu verhindern, wird wiederum ein Außenschlauch 4 in der erörterten Weise aufgebracht.

Eine weitere Ausführungsform eines Katheters 1''' ist schließlich in den Fig. 4 und 5 dargestellt. Im Innen- und Außenschlauch 3, 4 unterscheidet sich diese Ausführungsform nicht von der gemäß Fig. 1. Insoweit wird auf die dortigen Ausführungen verwiesen.

Das Profilteil 5''' ist einstückig aus einem harten Kunststoff-Material, wie etwa Polycarbonat oder Polyimid, oder auch aus dünnem Metallmaterial, wie Edelstahl oder Nitinol, hergestellt und weist auf den Teillängen t1, t2 und t4 parallel mit Abstand zueinander verlaufende Peripherstege 9 auf. Diese sind als Teilringe ausgebildet, deren Enden 11 jeweils durch einen axial durchgehenden Axialsteg 8, 8' miteinander verbunden sind. Zwischen den beiden Axialstegen 8, 8' bleibt ein Spalt 12, der im Katheter 1''' als Sollbruchstelle für dessen Auftrennen zum Abnehmen von der eingeführten Elektrode dient.

Auch bei der in Fig. 4 dargestellten Ausführungsform sind die Öffnungen 7 und insbesondere Peripherstege 9 auf den verschiedenen Teillängen t1 bis t4 unterschiedlich gestaltet. In der zum distalen Ende hin angeordneten Teillänge t1 sind relativ dünne Peripherstege 9 vorgesehen, sodass dort der Katheter relativ flexibel ist. In der in proximaler Richtung sich anschließenden Teillänge t2 sind die Peripherstege 9 deutlich verbreitert, wodurch die Flexibilität abnimmt.

Eine Besonderheit zeigen die Axialstege 8, 8' im Bereich der Teillänge t3. Dort weisen die Axialstege 8, 8' in Peripherrichtung P weisende Fortsätze 13, 13' auf, die so versetzt zueinander angeordnet sind, dass sie unter Bildung eines mäanderförmigen Spaltabschnittes 12' miteinander kämmen. In diesem Bereich ist der Katheter 1''' dadurch torsionsstabil.

Auf der Teillänge t4 sind relativ dünne Peripherstege 9 durch in Axialrichtung breite Öffnungen 7 voneinander getrennt, sodass der Katheter dort sehr flexibel in der durch den dortigen Spalt 12 verlaufenden Mittelebene ist.

Es schließt sich eine Teillänge t5 an, in der keine Öffnungen außer Spalt 12 vorgesehen sind. Dadurch ist dort der Katheter 1''' sehr steif.

In Fig. 5 ist das Profilteil 5''' auf seiner Teillänge t1 ohne Innen- und Außenschlauch dargestellt.

Schließlich ist zu erwähnen, dass die Profilteile aus einem Material hergestellt werden können, das für MR-Sichtgeräte kompatibel beziehungsweise in Ultraschall- oder Röntgen-Geräten detektierbar ist.

## Patentansprüche

1. Katheter, insbesondere zur Einführung von Herzschrittmacher- oder ICD-Elektroden in einen Patientenkörper, umfassend
- eine Katheterwand (2), die mindestens ein Lumen (6, 6') umgrenzt, und
- ein in der Katheterwand (2) befindliches Profilteil (5, 5', 5", 5"'), welches zumindest auf einer Teillänge (t1 bis t5) als Gitterprofil (5, 5', 5"') mit Axial- und/oder Peripherstegen (8, 9) ausgebildet ist, um den Katheter (1, 1', 1 ", 1"') bezüglich seiner Torsions-, Biege-, Kompressibilitäts- und/oder Knicksteifigkeit zu stabilisieren und bezüglich dessen mechanischer Eigenschaften abzustimmen,
**dadurch gekennzeichnet, dass**
das Profilteil (5, 5', 5", 5"') durch variable Profilierung auf unterschiedlichen Teillängen (t1 bis t5) oder durch einen variablen Querschnitt der Axial- und/oder Peripherstege (8, 9) in Axial- und/oder Peripherrichtung (A, P) hinsichtlich der Schlitzbarkeits-Eigenschaften des Katheters (1, 1', 1"') derart variabel ist, dass eine Sollbruchstelle entsteht.

2. Katheter nach Anspruch 1, **dadurch gekennzeichnet, dass** die Sollbruchstelle zum Auftrennen des Katheters (1, 1', 1") durch sehr dünne und kurze Peripherstege (9) zwischen zwei Reihen von Öffnungen (7) gebildet ist.

3. Katheter nach Anspruch 1 oder 2, **dadurch gekennzeichnet, dass** das Gitterprofilteil (5"') teilringförmige Peripherstege (9) aufweist, die an ihren Enden durch jeweils einen axial durchgehenden Axialsteg (8, 8') verbunden sind.

4. Katheter nach Anspruch 3, **dadurch gekennzeichnet, dass** die axial durchgehenden Axialstege (8, 8') einen axial durchgehenden Spalt (12) bilden, welcher als Sollbruchstelle für das Auftrennen des Katheters (1"') dient.

5. Katheter nach einem der vorgenannten Ansprüche, **dadurch gekennzeichnet, dass** das Profilteil (5, 5"') durch Schneiden oder Ätzen aus einem Schlauch- oder Folienteil aus Metall- oder Kunststoffmaterial hergestellt ist.

6. Katheter nach einem der Ansprüche 1 bis 5. **dadurch gekennzeichnet, dass** die Stege (8, 9) des Profilteils (5") durch strahlungsinduziertes oder chemisches Vernetzen eines aushärtbaren Kunststoff-Schlauchteils oder einer aushärtbaren Beschichtung hergestellt sind.

7. Katheter nach einem der Ansprüche 1 bis 5, **dadurch gekennzeichnet, dass** das Profilteil (5, 5', 5"') durch ein Heißverformungsverfahren, insbesondere durch Spritzgiessen, Extrusion oder dergleichen hergestellt ist.

8. Katheter nach einem der vorgenannten Ansprüche, **dadurch gekennzeichnet, dass** das Profilteil (5, 5', 5", 5"') in die Katheterwand (2) eingebettet, insbesondere eingespritzt ist.

9. Katheter nach einem der Ansprüche 1 bis 7, **dadurch gekennzeichnet, dass** das Profilteil (5, 5", 5"') zwischen einem Innenschlauch (3) und einem Außenschlauch (4) der Katheterwand (2) eingebettet ist.

## Claims

1. A catheter, in particular for the insertion of cardiac pacemaker or ICD electrodes into a patient body, comprising
- a catheter wall (2), which circumscribes at least one lumen (6, 6'), and
- a profile part (5, 5', 5", 5"'), located in the catheter wall (2), which is implemented on at least one partial length (t1 through t5) as a lattice profile (5, 5', 5"') having axial and/or peripheral webs (8, 9) to stabilize the catheter (1, 1', 1", 1"') in regard to its torsion, bending, compressibility, and/or buckling rigidity and adapt it in regard to its mechanical properties,
**characterized in that** the profile part (5, 5', 5", 5"') is variable in regard to the properties of the catheter (1, 1', 1"') for its ability to be slit by variable profiling on different partial lengths (t1 through t5) or by a variable cross-section of the axial and/or peripheral webs (8, 9) in the axial and/or peripheral directions (A, P) in such a way that an intended breakpoint arises.

2. The catheter according to Claim 1, **characterized in that** the intended breakpoint for breaking off the catheter (1, 1', 1"') is formed by very thin and short peripheral webs (9) between two rows of openings (7).

3. The catheter according to Claim 1 or 2, **characterized in that** the lattice profile part (5"') has peripheral webs (9) in the form of partial rings which are each connected on their ends by an axially continuous axial web (8, 8').

4. The catheter according to Claim 3, **characterized in that** the axially continuous axial webs (8, 8') form an axially continuous gap (12), which is used as an intended breakpoint for breaking off the catheter (1 "').

5. The catheter according to one of the preceding claims, **characterized in that** the profile part (5, 5"') is produced by cutting or etching from a tube or film part made of metal or plastic material.

6. The catheter according to one of Claims 1 through 5, **characterized in that** the webs (8, 9) of the profile part (5") are produced by radiation-induced or chemical cross-linking of a curable plastic tube part or a curable coating.

7. The catheter according to one of Claims 1 through 5, **characterized in that** the profile part (5, 5', 5"') is produced by a hot molding method, in particular by injection molding, extrusion, or the like.

8. The catheter according to one of the preceding claims, **characterized in that** the profile part (5, 5', 5", 5"') is embedded, in particular covered by injection, in the catheter wall (2).

9. The catheter according to one of Claims 1 through 7, **characterized in that** the profile part (5, 5", 5"') is embedded between an inner tube (3) and outer tube (4) of the catheter wall (2).

## Revendications

1. Cathéter, en particulier pour l'introduction d'électrodes de pacemaker ou d'électrodes ICD dans un corps de patient, comprenant :
- une paroi de cathéter (2), qui délimite au moins un lumen (6, 6'), et
- une partie de profilé (5, 5', 5", 5"') se trouvant dans la paroi de cathéter (2), qui est réalisée au moins sur une longueur partielle, (t1 à t5) sous forme de profilé de grille (5, 5', 5"') avec des nervures axiales et/ou périphériques (8, 9), afin de stabiliser le cathéter (1, 1', 1", 1"') par rapport à sa rigidité à la torsion, à la flexion, à la compassivité et/ou au flambage et de l'adapter par rapport à ses propriétés mécaniques, **caractérisé en ce que**
la partie de profilé (5, 5', 5", 5"') peut être modifiée par un profilage variable sur différentes longueurs partielles (t1 à t5) ou par une section variable des nervures axiales et/ou périphériques (8, 9), dans le sens axial et/ou périphérique (A, P) en ce qui concerne les propriétés d'entaillabilité du cathéter (1, 1', 1"') de telle sorte qu'il se forme un point de rupture théorique.

2. Cathéter selon la revendication 1, **caractérisé en ce que** le point de rupture théorique pour la séparation du cathéter (1, 1', 1") est formé par des nervures périphériques (9) très minces et courtes entre deux rangées d'ouvertures (7).

3. Cathéter selon la revendication 1 ou 2, **caractérisé en ce que** la partie de profilé de grille (5"') présente des nervures périphériques (9) en forme de bague partiel, qui sont reliées chacune par leurs extrémités par une nervure axiale (8, 8') continue dans le sens axial.

4. Cathéter selon la revendication 3, **caractérisé en ce que** les nervures axiales (8, 8') continues dans le sens axial forment une fente continue dans le sens axial, qui sert de point de rupture théorique pour la séparation du cathéter (1 "').

5. Cathéter selon l'une quelconque des revendications précédentes, **caractérisé en ce que** la partie de profilé (5, 5"') est fabriquée par découpage ou décapage dans une partie de flexible ou une partie de feuille à base de matériau métallique ou de matière plastique.

6. Cathéter selon l'une quelconque des revendications 1 à 5, **caractérisé en ce que** les nervures (8, 9) de la partie de profilé (5") sont fabriquées par réticulation induite par rayonnement ou chimique d'une partie de flexible en plastique durcissable ou d'un revêtement durcissable.

7. Cathéter selon l'une quelconque des revendications 1 à 5, **caractérisé en ce que** la partie de profilé (5, 5', 5"') est fabriquée par un procédé de déformation à chaud, en particulier par moulage par injection, extrusion ou similaires.

8. Cathéter selon l'une quelconque des revendications précédentes, **caractérisé en ce que** la partie de profilé (5, 5', 5", 5"') est enfoncée, en particulier injectée, dans une paroi de cathéter (2).

9. Cathéter selon l'une quelconque des revendications 1 à 7, **caractérisé en ce que** la partie de profilé (5, 5", 5"') est enfoncée entre un flexible intérieur (3) et un flexible extérieur (4) de la paroi de cathéter (2).
